# EUROPEAN PATENT APPLICATION

(11) **EP 4 520 795 A1**
(43) Date of publication of application: **12.03.2025**
(21) Application number: 24195295.1
(22) Date of filing: 20.08.2024
(51) Int. Cl.: C09D 5/16

(54) **ANTIFOULING MEMBER, MEMBER FOR IN-VITRO DIAGNOSTIC APPARATUS, AND METHOD OF PRODUCING ANTIFOULING MEMBER**

(30) Priority: 06.09.2023 JP 2023144815
(71) Applicant: CANON KABUSHIKI KAISHA, Tokyo 146-8501 (JP)
(72) Inventor: MASUMOTO, Akane, Tokyo (JP); KOSAKI, Yusuke, Tokyo (JP); UESUGI, Tomoya, Tokyo (JP); TAKAHATA, Nozomu, Tokyo (JP)
(74) Representative: WESER & Kollegen Patentanwälte PartmbB

(57) **Abstract**

Provided is an antifouling member that can achieve both low protein adsorptivity and durability. The antifouling member has a membrane formed on a base material. The membrane contains a condensate of a hydrolysate of a silane coupling agent or a condensate of a phosphonic acid derivative. The membrane does not contain a halogen atom. The membrane has a zeta potential of -10.0 mV to 10.0 mV in water. The antifouling member has a value of B/A of 2.0 to 5.0, where A represents a ratio of an amount of C atom to an amount of atom of an element X contained in a surface of the base material in a largest amount in XPS analysis of the base material, and B represents a ratio of an amount of C atom to an amount of atom of the element X in XPS analysis of the membrane on the base material.

## Description

### BACKGROUND OF THE INVENTION

### Field of the Invention

The present invention relates to an antifouling member, a member for an in-vitro diagnostic apparatus, and a method of producing an antifouling member.

### Description of the Related Art

Unlike single-use equipment, such as a pacemaker, a catheter, and a guidewire, members that can be used a plurality of times, such as forceps, an endoscope, a dental drill, and a member for an in-vitro diagnostic apparatus, are reused after appropriate cleaning and sterilization treatment. However, in order to ensure safety, a burden of cleaning is significant, and hence a member having an antifouling property that can be used a plurality of times is desired in order to reduce the burden.

In a member for an in-vitro diagnostic apparatus for biochemical analysis, etc. among those that can be used a plurality of times, an adsorption of very small amount of protein causes a decrease in detection sensitivity and a decrease in reproducibility. A conventional member for an in-vitro diagnostic apparatus has been cleaned with an alkali between specimens to reduce an influence of protein adsorption. However, in recent years, sensitivity of the in-vitro diagnostic apparatus has been advanced, and hence causes concern that the influence of protein adsorption may not be eliminated completely by conventional cleaning alone. Thus, an antifouling coating for the member for an in-vitro diagnostic apparatus is desired.

Under such a situation, there have been widely proposed surface treatment agents that can impart surface hydrophilicity, biocompatibility, an antithrombotic property, etc. through treatment of surfaces of various members with polymerized products of betaine monomers.

In Japanese Patent No. 6,154,370, there is proposed a method involving treating a medical device, such as a catheter or a guidewire, with a silane coupling agent having a vinyl group and then polymerizing a sulfobetaine monomer for the purpose of improving and maintaining slidability.

In addition, in Japanese Patent No. 5,349,873, there are proposed a medical material in which a polymer brush using a carboxybetaine monomer is formed and a medical instrument using the medical material.

### SUMMARY OF THE INVENTION

However, according to the investigations made by the inventors of the present invention, methods described in Japanese Patent No. 6,154,370 and Japanese Patent No. 5,349,873 each have a problem in durability to an alkali cleaning solution used for cleaning an antifouling member.

Thus, an object of the present invention is to provide an antifouling member that can achieve both low protein adsorptivity and durability. In addition, another object of the present invention is to provide a method of producing an antifouling member that can achieve both low protein adsorptivity and durability.

The above-mentioned objects are achieved by the present invention described below. That is, according to the present invention, there is provided an antifouling member including a membrane formed on a base material, wherein the membrane contains a condensate of a hydrolysate of a silane coupling agent or a condensate of a phosphonic acid derivative, wherein the membrane does not contain a halogen atom, wherein the membrane has a zeta potential of -10.0 mV or more and 10.0 mV or less in water having a pH of 7, and wherein the antifouling member has a value of B/A of 2.0 or more and 5.0 or less, where A represents a ratio of an amount of C atom (atomic%) to an amount of atom of an element X (atomic%) contained in a surface of the base material in a largest amount in X-ray photoelectron spectroscopy analysis (ESCA measurement) of the base material, and B represents a ratio of an amount of C atom (atomic%) to an amount of atom of the element X (atomic%) in X-ray photoelectron spectroscopy analysis (ESCA measurement) of the membrane on the base material.

Further features of the present invention will become apparent from the following description of exemplary embodiments with reference to the attached drawings.

### BRIEF DESCRIPTION OF THE DRAWINGS

FIG. 1 is an exemplary schematic view for illustrating an application example of the present invention.
FIG. 2 is another exemplary schematic view for illustrating an application example of the present invention.

### DESCRIPTION OF THE EMBODIMENTS

Now, the present invention is described in detail by way of exemplary embodiments.

### [First Embodiment]

A first embodiment is directed to an antifouling member.

An antifouling member of the present invention is an antifouling member including a membrane formed on a base material, wherein the membrane contains a condensate of a hydrolysate of a silane coupling agent or a condensate of a phosphonic acid derivative, wherein the membrane does not contain a halogen atom, wherein the membrane has a zeta potential of -10.0 mV or more and 10.0 mV or less in water having a pH of 7, and wherein the antifouling member has a value of B/A of 2.0 or more and 5.0 or less, where A represents a ratio of an amount of C (carbon) atom (atomic%) to an amount of atom of an element X (atomic%) contained in a surface of the base material in a largest amount in X-ray photoelectron spectroscopy analysis (ESCA measurement) of the base material, and B represents a ratio of an amount of C atom (atomic%) to an amount of atom of the element X (atomic%) in X-ray photoelectron spectroscopy analysis (Electron Spectroscopy for Chemical Analysis, ESCA measurement) of the membrane on the base material.

The antifouling member satisfying the above-mentioned constitution can achieve both low protein adsorptivity and durability. The antifouling member of the present invention is an antifouling member having a membrane formed on a base material. A conventional antifouling member has been obtained by polymerizing a betaine monomer with a silane coupling agent having a vinyl group. However, for those which are desired to have durability to an alkali cleaning solution, such as an antifouling member, an effect has not been sufficient. The inventors have made extensive investigations, and as a result, have found that durability to an alkali cleaning solution can be exhibited by controlling two parameters within specific ranges.

In the antifouling member of the present invention, a membrane does not contain a halogen atom. One of parameters indicating that the membrane does not contain a halogen atom is that a ratio of an ion amount of halogen atom to a total ion amount is 0.02 or less in TOF-SIMS measurement of the membrane. In the TOF-SIMS measurement, information on elements or molecular species that are present at a depth of several nanometers or less from a sample surface can be obtained with significantly high detection sensitivity. Thus, in the TOF-SIMS measurement of the membrane, information on elements or molecular species that are present on the outermost surface of the membrane is obtained. The ratio of the ion amount of halogen atom to the total ion amount of 0.02 or less indicates the presence of a halogen on the outermost surface of the membrane. When the ratio of the ion amount of halogen atom to the total ion amount is 0.02 or less, satisfactory durability can be exhibited. The ratio of the ion amount of halogen atom to the total ion amount of 0.02 or less is only a guideline, and it is appropriate that the ratio of the halogen atom ion amount to the total ion amount be at a background level (level at which a halogen atom is not detected) in the TOF-SIMS measurement in a sense that a halogen atom is not clearly detected in the membrane in the TOF-SIMS measurement.

The inventors have conceived the reason for the foregoing as described below.

When the membrane in the above-mentioned configuration contains a halogen, the halogen is present in a state of being bonded to carbon. Here, the main component of an alkaline cleaning solution is water, and hence it is conceived that the halogen and the carbon to which the halogen is bonded are polarized to be more susceptible to solvation by water. In other words, it is conceived that the membrane containing a halogen on the outermost surface has a higher affinity for the alkaline cleaning solution than a membrane which does not contain a halogen. In general, a condensate of a hydrolysate of a silane coupling agent or a condensate of a phosphonic acid derivative is known to be eluted in an alkaline solution. Thus, it is conceived that, because of the high affinity for the alkaline cleaning solution, the membrane containing a halogen undergoes progressive membrane deterioration due to the elution of the condensate of the hydrolysate of the silane coupling agent or the condensate of the phosphonic acid derivative, and durability becomes insufficient. Accordingly, in order to exhibit sufficient durability, it is important, for example, that the ratio of the halogen atom ion amount to the total ion amount be 0.02 or less.

The antifouling member of the present invention has a value of B/A of 2.0 or more and 5.0 or less, where A represents a ratio of an amount of C atom (atomic%) to an amount of atom of an element X contained in a surface of a base material in a largest amount in X-ray photoelectron spectroscopy analysis (ESCA measurement) of the base material, and B represents a ratio of an amount of C atom (atomic%) to an atom amount of the element X (atomic%) in X-ray photoelectron spectroscopy analysis (ESCA measurement) of the membrane on the base material. In the ESCA measurement, photoelectrons generated in a region from the outermost surface to about 10 nm are detected. The value of B/A provides information on the density and thickness of the membrane. When the value is 2.0 or more and 5.0 or less, good low protein adsorptivity and durability are exhibited. When the value is less than 2.0, hydrophilicity becomes insufficient due to insufficient density and thickness of the membrane, and low protein adsorptivity is not sufficiently exhibited. Meanwhile, when the value is more than 5.0, the thickness of the membrane becomes large, and hydrophilicity is sufficiently exhibited, with a result that low protein adsorptivity is exhibited. However, an affinity for an alkali cleaning solution becomes too high, and the membrane deterioration caused by the elution of the condensate of the hydrolysate of the silane coupling agent or the condensate of the phosphonic acid derivative progresses, resulting in insufficient durability. Thus, it is important that the value of B/A be 2.0 or more and 5.0 or less.

In addition, the antifouling member of the present invention is a member that is reused after appropriate cleaning and sterilization treatment, such as forceps, an endoscope, a dental drill, or a member for an in-vitro diagnostic apparatus. Among such antifouling members, the member for an in-vitro diagnostic apparatus is preferred because the member for an in-vitro diagnostic apparatus is liable to be influenced by the adsorption of very small amount of protein and effects of the present invention can be effectively exhibited.

In addition, in the antifouling member of the present invention, the membrane contains a condensate of a hydrolysate of a silane coupling agent or a condensate of a phosphonic acid derivative. It is preferred that the condensate of the hydrolysate of the silane coupling agent or the condensate of the phosphonic acid derivative form a covalent bond with the base material and have a vinyl group in a molecule. No particular limitation is not provided as long as these conditions are satisfied, and a silane coupling agent or a conventionally known phosphonic acid derivative that has hitherto been known may be used. In the antifouling member of the present invention, from a viewpoint of durability, the silane coupling agent is preferably at least one kind selected from the group consisting of 3-(methacryloyloxy)propyltrimethoxysilane; 3-(acryloyloxy)propyltrimethoxysilane; 3-(methacryloyloxy)propyltriethoxysilane; 3-(acryloyloxy)propyltriethoxysilane; vinyltrimethoxysilane; triethoxyvinylsilane; allyltrimethoxysilane; allyltriethoxysilane; and (triethoxysilyl)methyl methacrylate. In addition, in the antifouling member of the present invention, from a viewpoint of durability, the phosphonic acid derivative is preferably at least one kind selected from the group consisting of: 2-methacryloyloxyethyl acid phosphate; 2-acryloyloxyethyl acid phosphate; 2-(methacryloyloxy)ethyl phosphate; and acid phosphoxy polyethylene glycol monomethacrylate. Among those, 3-(methacryloyloxy)propyltrimethoxysilane and 2-methacryloyloxyethyl acid phosphate are preferred from a viewpoint of achieving both low protein adsorptivity and durability.

In addition, in the antifouling member of the present invention, a zeta potential at a pH of 7 is -10.0 mV or more and 10.0 mV or less. A mechanism of protein adsorption to the base material, etc. is based on two interactions: an electrostatic interaction and a hydrophobic interaction. Thus, it is important to control the zeta potential at a pH of 7 within the above-mentioned range in order to suppress the protein adsorption caused by the electrostatic interaction. Any material may be used as long as the zeta potential can be controlled within the above-mentioned range, but it is preferred that the zeta potential be controlled by any one or a combination of two or more of the following three structural features.

A first structural feature is that, in the antifouling member of the present invention, the membrane preferably contains a copolymer of a monomer having polarity opposite to polarity of the base material, a hydrophilic monomer, and a cross-linking agent. With this structural feature, a surface charge of the base material is cancelled by a monomer having polarity opposite to polarity of the base material. In addition, due to cross-linking, a base material shielding property exhibited by the membrane is increased to enable the membrane to be less influenced by the surface charge of the base material. Further, when the hydrophilic monomer is also copolymerized, the low protein adsorptivity derived from the hydrophobic interaction can also be realized. It is preferred that a copolymerization ratio in the copolymer of the monomer having polarity opposite to polarity of the base material, the hydrophilic monomer, and the cross-linking agent are adjusted so that the zeta potential at a pH of 7 is -10.0 mV or more and 10.0 mV or less.

Here, in the antifouling member of the present invention, any monomer may be used as the monomer having polarity opposite to polarity of the base material, and an amine monomer is preferred among them. In the antifouling member of the present invention, from a viewpoint of reactivity, the amine monomer is preferably at least one kind selected from the group consisting of 2-(dimethylamino)ethyl methacrylate; 2-(dimethylamino)ethyl acrylate; 2-(diethylamino)ethyl methacrylate; 2-(diisopropylamino)ethyl methacrylate; and 2-(tert-butylamino)ethyl methacrylate. Examples of the amine monomer include 2-(dimethylamino)ethyl methacrylate, 2-(dimethylamino)ethyl acrylate, 2-(diethylamino)ethyl methacrylate, 2-(diisopropylamino)ethyl methacrylate, and 2-(tert-butylamino)ethyl methacrylate. Among those, 2-(dimethylamino)ethyl methacrylate and 2-(diethylamino)ethyl methacrylate are more preferred from a viewpoint of reactivity.

In the antifouling member of the present invention, the hydrophilic monomer is preferably electrically neutral. In the antifouling member of the present invention, the hydrophilic monomer is preferably at least one kind selected from the group consisting of: 2-hydroxyethyl methacrylate; N-vinyl-2-pyrrolidone; and polyethylene glycol monomethyl ether methacrylate. Examples of the hydrophilic monomer include 2-hydroxyethyl methacrylate, N-vinyl-2-pyrrolidone, polyethylene glycol monomethyl ether methacrylate, 2,3-dihydroxypropyl methacrylate, 2-hydroxyethyl acrylate, hydroxypropyl methacrylate, and 2-hydroxy-3-phenoxypropyl 2-methylpropenoate. Among those, 2-hydroxyethyl methacrylate and poly(ethylene glycol) monomethyl ether methacrylate are more preferred from a viewpoint of low protein adsorptivity.

In the antifouling member of the present invention, the cross-linking agent is preferably at least one kind selected from the group consisting of: ethylene glycol diacrylate; ethylene glycol dimethacrylate; polyethylene glycol diacrylate; polyethylene glycol dimethacrylate; and 1,6-hexanediol diacrylate. Thus, a gap between the polymers can be filled, and low protein adsorptivity can be achieved. Examples of the cross-linking agent include ethylene glycol diacrylate, ethylene glycol dimethacrylate, polyethylene glycol diacrylate, polyethylene glycol dimethacrylate, 1,6-hexanediol diacrylate, divinylbenzene, neopentyl glycol diacrylate, 3-methyl-1,5-pentanediol diacrylate, 1,9-nonanediol diacrylate, an acrylic acid adduct of neopentyl glycol hydroxypivalate, and 2-hydroxy-3-acryloyloxypropyl methacrylate. Among those, polyethylene glycol diacrylate and 1,6-hexanediol diacrylate are preferred from a viewpoint of low protein adsorptivity.

In addition, the second structural feature is that, in the antifouling member of the present invention, the membrane preferably contains a polymer of a betaine monomer. The polymer of a betaine monomer has a positive charge and a negative charge in a molecule. Thus, the chains of the polymer attract each other by the electrostatic interaction to cause shielding action between the chains, and the zeta potential at a pH of 7 can be controlled to -10.0 mV or more and 10.0 mV or less. In the antifouling member of the present invention, the betaine monomer is preferably at least one kind selected from the group consisting of 2-(methacryloyloxy)ethyl 2-(trimethylammonio)ethyl phosphate; 2- [[2-(methacryloyloxy)ethyl] dimethylammonio] acetate; 3-[[2-(methacryloyloxy)ethyl]dimethylammonio]propionate; 3-[(3-acrylamidopropyl)dimethylammonio]propanoate; 3-[[2-(methacryloyloxy)ethyldimethylammonio]propane-1-sulfonate; 4-[[2-(methacryloyloxy)ethyl]dimethylammonio]butane-1-sulfonate; 3-[[2-(acryloyloxy)ethyl]dimethylammonio]propane-1-sulfonate; 3-[(3-methacrylamidopropyl)dimethylammonio]propane-1-sulfonate; and 3-[(3-acrylamidopropyl)dimethylammonio]propane-1-sulfonate. Among those, 3-[[2-(methacryloyloxy)ethyl]dimethylammonio]propionate, 2-(methacryloyloxy)ethyl 2-(trimethylammonio)ethyl phosphate, and 3-[[2-(methacryloyloxy)ethyl]dimethylammonio]propane-1-sulfonate are more preferred from a viewpoint of low protein adsorptivity.

In addition, the third structural feature is that, in the antifouling member of the present invention, the membrane preferably contains the polymer of the betaine monomer and a polymer of the hydrophilic monomer, or a copolymer of the betaine monomer and the hydrophilic monomer. The membrane may have a configuration further containing a polymer of the hydrophilic monomer or the copolymer of the betaine monomer and the hydrophilic monomer on the polymer of the betaine monomer. Thus, the zeta potential at a pH of 7 can be controlled to -10.0 mV or more and 10.0 mV or less, and low protein adsorptivity can be achieved.

In the antifouling member of the present invention, a material of the base material preferably includes stainless steel or glass. Thus, the antifouling member of the present invention can be used as a member for an in-vitro diagnostic apparatus described later.

In the antifouling member of the present invention, the membrane preferably includes a charge adjustment site having polarity opposite to polarity of the base material and a hydrophilic site. As used herein, the charge adjustment site and the hydrophilic site may be present in separate layers or in the same layer. The charge adjustment site is, for example, a site formed of the monomer having polarity opposite to polarity of the base material or a site formed of the betaine monomer. The hydrophilic site is, for example, a site formed of the hydrophilic monomer or a site formed of the betaine monomer.

In addition, in the antifouling member of the present invention, it is preferred that a bonding site which is bonded to the base material is represented by the following formula (1-1) or the following formula (1-2), that the charge adjustment site is a copolymerized product of monomers represented by the following formula (2-1), the following formula (2-2), and the following formula (2-3), or is represented by the following formula (2-4), and that the hydrophilic site be represented by the following formula (3-1) or the following formula (3-2): 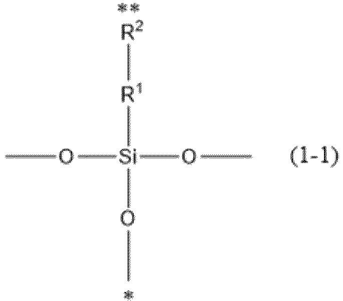 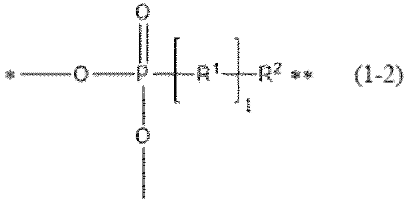 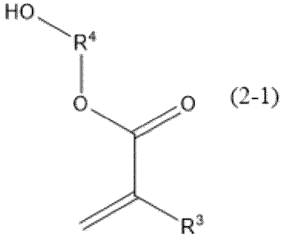 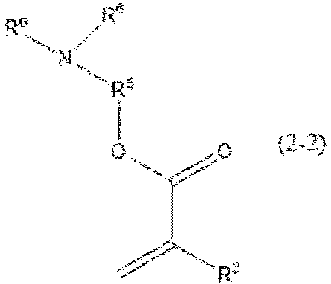 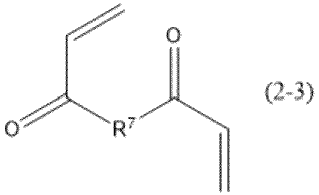 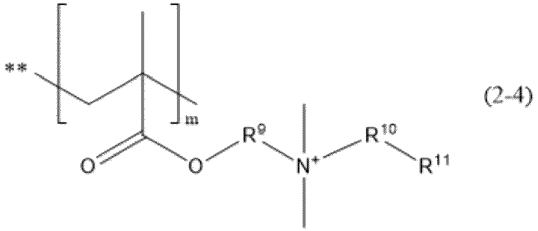 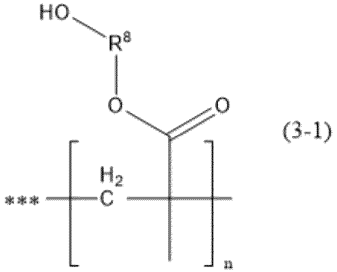 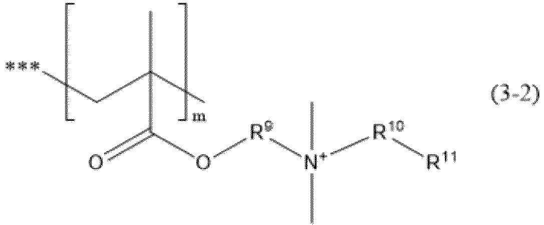 in the formula (1-1) and the formula (1-2), "*" represents a bonding position to the base material, "**" represents a bonding position to the charge adjustment site, R¹ represents any one of an alkylene group or an oxyalkylene group, and R² represents any one of CH=CH (vinylene group), CH=C(CH₃) (isopropenylene group), HC=CH=C(=O)-O (acryloyloxy group), HC=C(CH₃)-C(=O)-O (methacryloyloxy group), or HC=C(CH₃)-C(=O)-NH (methacryloylamino group), and "1" satisfies 1≤1≤6; in the formula (2-1), the formula (2-2), and the formula (2-3), R³ represents hydrogen or a methyl group, R⁴, R⁵, and R⁸ each independently represent an alkylene group having 1 to 6 carbon atoms, R⁶ represents hydrogen or an alkyl group having 1 to 3 carbon atoms, and R⁷ represents an oxyalkylene group having 1 to 3 carbon atoms; in the formula (2-4), "**" represents a bonding position to the bonding site, R⁹ and R¹⁰ each independently represent any one of an alkylene group or an oxyalkylene group, R¹¹ represents -COO⁻, and "m" satisfies 4≤m≤1,000; and in the formula (3-1) and the formula (3-2), "***" represents a bonding position to the charge adjustment site, R⁸ represents an alkylene group having 1 to 6 carbon atoms, "n" satisfies 4≤n≤1,000, R⁹ and R¹⁰ each independently represent any one of an alkylene group or an oxyalkylene group, R¹¹ represents -COO⁻, and "m" satisfies 4≤m≤1,000.

### <Method of measuring Zeta Potential of Membrane>

The zeta potential of the membrane may be measured by an electrophoretic light scattering method through use of a zeta potential measurement system ELSZ-2000 (manufactured by Otsuka Electronics Co., Ltd.). The membrane is attached to the upper surface of a cell of a cell unit for a flat plate sample (manufactured by Otsuka Electronics Co., Ltd.), and the cell is filled with a 10 mM sodium chloride aqueous solution having a pH of 7 in which monitor particles (manufactured by Otsuka Electronics Co., Ltd.) are dispersed. The monitor particles are electrophoresed, and the electrical mobility of the monitor particles is measured. The zeta potential (mV) of the membrane at a pH of 7 may be calculated by analyzing the data on the resultant electrical mobility by the Mori-Okamoto equation and the Smoluchowski equation.

### <TOF-SIMS Measurement Method for Membrane>

TOF-SIMS (manufactured by ULVAC-PHI, Inc., nanoTOF II) is used to detect a halogen present on the surface of the membrane. The analysis conditions are as described below.

Sample adjustment: A base material having a membrane formed thereon is caused to adhere to an indium sheet.
Sample pretreatment: None
Primary ion: Bi₃⁺⁺
Accelerating voltage: 30 kV
Charge neutralization mode: On
Measurement mode: Negative
Raster size: 300 µm
Integrated time period: 180 seconds

The ratio of the halogen atom ion amount to the total ion amount of the membrane may be determined from ion amounts obtained by performing the above-mentioned measurement.

### <X-ray Photoelectron Spectroscopy analysis (ESCA Measurement) Method>

The measurement device and measurement conditions are as described below.
·Measurement device: X-ray photoelectron spectrometer: Quantum 2000 (manufactured by ULVAC-PHI, Inc.)
·X-ray source: Monochrome Al Kα
·X-ray setting: 100 µmϕ (25 W (15 KV))
·Photoelectron extraction angle: 45 degrees
·Neutralization condition: Combination use of a neutralization gun and an ion gun
·Analysis region: 300 µm×200 µm
·Pass energy: 58.70 eV
·Step size: 0.125 eV
·Analysis software: MaltiPak (ULVAC-PHI, Inc.)

In the measuring by XPS method, qualitative analysis in an entire element range is first performed by Survey analysis, and then quantitative analysis by narrow-scanning is performed for each detected element at a number of scans shown in Table 1.

**Table 1**

| C1s | N1s | O1s | Si2p | Cr2p3 | Fe2p3 | P2p | Br3d | Ca2p | S | Na | Br |
|---|---|---|---|---|---|---|---|---|---|---|---|
| 20 | 40 | 10 | 40 | 40 | 40 | 40 | 20 | 20 | 40 | 20 | 20 |

In quantitative values obtained in the ESCA measurement of the base material, a ratio of an amount of C atom (atomic%) to an amount of atom of an element X (atomic%) contained in the surface of the base material in the largest amount is represented by A, and in the quantitative values obtained in the ESCA measurement of the membrane on the base material, a ratio of an amount of C atom (atomic%) to an amount of atom of the element X (atomic%) is represented by B. Thus, a value of B/A can be determined.

### [Second Embodiment]

A second embodiment is directed to a method of producing an antifouling member. Each constitution of the antifouling member is as described above, and hence description thereof is omitted.

In addition, the present invention relates to a method of producing a member having a membrane formed on a surface of a base material. Three examples are given as preferred examples of the production method. A first example of these examples is described.

The method of producing an antifouling member of the present invention (the first example) is a method of producing an antifouling member having a membrane formed on a surface of a base material, the method including: a bonding step of bonding a hydrolysate of a silane coupling agent or a condensate of a phosphonic acid derivative to the surface of the base material; a charge adjustment site forming step of copolymerizing a monomer having polarity opposite to polarity of the base material, a hydrophilic monomer, and a cross-linking agent in presence of a polymerization initiator after the bonding step; and a hydrophilic site forming step of adding and polymerizing a hydrophilic monomer to form a hydrophilic site after the charge adjustment site forming step.

The above-mentioned kinds of silane coupling agents may each be used as the silane coupling agent. Hydrolysis of a silane coupling agent may be performed by a known method. Optimum pH for the hydrolysis varies depending on a kind of the silane coupling agent, but a pH at which a condensation speed is slow is preferably selected. For example, in the case of 3-(methacryloyloxy)propyltrimethoxysilane, it is preferred to perform hydrolysis in an aqueous solution at a pH of 4±0.5 because its condensation can be suppressed. In addition, a concentration of the silane coupling agent in hydrolyzing is preferably 0.5 wt% or more and 70.0 wt% or less. The concentration of the silane coupling agent is more preferably 0.5 wt% or more and 5.0 wt% or less, and when the concentration falls within such range, the condensation of a hydrolysate of the silane coupling agent can be effectively suppressed.

The bonding step of bonding the hydrolysate of the silane coupling agent to the base material is described. It is preferred that the base material is used after being subjected to ultrasonic cleaning through use of one kind or a plurality of kinds of alkalis, surfactants, or organic solvents to remove organic matter adhering to a surface. In addition, it is more preferred that the base material is used after being subjected to UV/O₃ cleaning for 1 minute or more and 60 minutes or less to remove organic matter adhering to the surface. The cleaned base material is immersed in an aqueous solution having a pH of 4±0.5 in which 0.5 wt% or more and 5.0 wt% or less of the hydrolysate of the silane coupling agent is dissolved for 30 seconds or more and 10 minutes or less. After that, a resultant is dried under an atmosphere of 10°C or more and 40°C or less for 30 seconds or more and 1,800 seconds or less. After drying, an excess hydrolysate of the silane coupling agent is removed by washing with water, and then a resultant is placed in an oven at 50°C or more and 150°C or less to be calcinated. Thus, the hydrolysate of the silane coupling agent is bonded to the base material to perform the bonding step.

In addition, the above-mentioned kinds of phosphonic acid derivatives may each be used as the phosphonic acid derivative. It is preferred that the phosphonic acid derivative is used after being dissolved in a solvent in which the phosphonic acid derivative is soluble. Preferred examples of the solvent include an alcohol, tetrahydrofuran, acetone, N-methylpyrrolidone, and dimethylformamide. Among those, an alcohol and tetrahydrofuran are more preferred because the alcohol and tetrahydrofuran enable the bonding step with respect to the base material to proceed suitably.

The bonding step of bonding the condensate of the phosphonic acid derivative to the base material is described. It is preferred that cleaning of the base material is performed in a same manner as in the bonding step using the silane coupling agent. 0.1 mmol/l or more and 50 mmol/l or less of a phosphonic acid derivative solution is prepared. Any solvent in which the phosphonic acid derivative is soluble may be used, and examples thereof include an alcohol, tetrahydrofuran, acetone, toluene, N-methylpyrrolidone, and dimethylformamide. Among those, an alcohol and tetrahydrofuran are more preferred. The cleaned base material is immersed in the prepared phosphonic acid derivative solution for 1 minute or more and 120 minutes or less. After that, the resultant is placed in an oven at 15°C or more and 150°C or less for 1 minute or more and 2,880 minutes or less. Then, ultrasonic cleaning is performed with tetrahydrofuran and methanol to remove a multilayer film. Thus, the condensate of the phosphonic acid derivative is bonded to the base material.

Next, the charge adjustment site forming step is described. The charge adjustment site forming step is performed by copolymerizing a monomer having polarity opposite to polarity of the base material, a hydrophilic monomer, and a cross-linking agent in the presence of a polymerization initiator and the base material having been subjected to the bonding step. No particular limitation on a copolymerization method is provided as long as the monomers can be copolymerized under a condition in which the polymerization initiator coexists with the monomers, but a method involving copolymerizing the monomers after the polymerization initiator is brought into contact with the base material having been subjected to the bonding step is preferred from a viewpoint of reactivity.

Any method may be used as a method of bringing the polymerization initiator into contact with the base material having been subjected to the bonding step, and a method involving placing the base material having been subjected to the bonding step in a reaction vessel, performing nitrogen purging, and then placing a liquid, in which the polymerization initiator is dissolved is added to a reaction solvent in which oxygen has been removed therefrom by nitrogen bubbling, in the reaction vessel, followed by stirring is preferred. After that, a monomer having polarity opposite to polarity of the base material, a hydrophilic monomer, and a cross-linking agent are dissolved in a reaction solvent, and nitrogen purging is performed. The resultant is placed in the reaction vessel containing the base material and the polymerization initiator, and copolymerization is performed by heating. Such copolymerization method is preferred. As the monomer having polarity opposite to polarity of the base material, the hydrophilic monomer, and the cross-linking agent, those described above may be used.

In addition, as a reaction solvent, water, an alcohol, tetrahydrofuran, acetone, N-methylpyrrolidone, dimethylformamide, etc. may be used alone or in combination thereof. Among those, water and an alcohol are preferred from a viewpoint of monomer solubility. In addition, a reaction temperature is preferably determined based on the 10-hour half-life temperature of the polymerization initiator to be used, but from viewpoints of the stability of the monomers to be used and stability of the copolymer, a reaction is performed preferably at 15°C or more and 90°C or less, more preferably at 40°C or less and 80°C or less. In addition, a reaction time (heating time) is preferably 10 minutes or more and 1,440 minutes or less, more preferably 30 minutes or more and 600 minutes or less.

The polymerization initiator is preferably 2,2'-azobis[N-(2-carboxyethyl)-2-methylpropionamidine] n-hydrate, which is common in the first example to a third example of the method of producing an antifouling member of the present invention.

In addition, examples of the polymerization initiator include azobisisobutyronitrile (AIBN), 2,2'-azobis[2-(2-imidazolin-2-yl)propane] dihydrochloride, 2,2'-azobis[2-(2-imidazolin-2-yl)propane]disulfate dihydrate, 2,2'-azobis(2-methylpropionamidine) dihydrochloride, 2,2'-azobis[N-(2-carboxyethyl)-2-methylpropionamidine] n-hydrate, 2,2'-azobis[2-(2-imidazolin-2-yl)propane], 2,2'-azobis(1-imino-1-pyrrolidino-2-methylpropane) dihydrochloride, 2,2'-azobis[2-methyl-N-(2-hydroxyethyl)propionamide], 2,2'-azobis(4-methoxy-2,4-dimethylvaleronitrile), 2,2'-azobis(2,4-dimethylvaleronitrile), dimethyl 2,2'-azobis(2-methylpropionate), 2,2'-azobis(2-methylbutyronitrile), 1,1'-azobis(cyclohexane-1-carbonitrile), 2,2'-azobis[N-(2-propenyl)-2-methylpropionamide], 1,1'-azobis(1-acetoxy-1-phenylethane), dimethyl 2,2'-azobisisobutylate, and derivatives thereof. Among those, 2,2'-azobis[N-(2-carboxyethyl)-2-methylpropionamidine] n-hydrate is preferred from a viewpoint of reactivity.

Next, the hydrophilic site forming step is described. This step is a step to be performed after the above-mentioned charge adjustment site forming step, and is a step of adding and polymerizing a hydrophilic monomer to form a hydrophilic site. As the hydrophilic monomer, those described above may be used. An addition and polymerization method may be performed by dissolving the hydrophilic monomer in a reaction solvent, performing nitrogen purging, and then placing a resultant in a reaction system subjected to the charge adjustment site forming step, followed by heating. A heating temperature may be set to a same temperature range as that of the charge adjustment site forming step and may be same as or different from that of the charge adjustment site forming step. In addition, a reaction solvent described in the charge adjustment site forming step may also be used as the reaction solvent, and the reaction solvent may be same as or different from that in the charge adjustment site forming step. A reaction time (heating time) is preferably 10 minutes or more and 1,440 minutes or less, more preferably 30 minutes or more and 600 minutes or less.

The membrane can be formed on the base material as described above, and cleaning may also be performed after the membrane is formed. Any method may be used as a cleaning method, and a method involving performing ultrasonic cleaning through use of a reaction solvent is preferred.

Next, a second example of the production method is described.

The method of producing an antifouling member of the present invention (the second example) is a method of producing an antifouling member having a membrane formed on a surface of a base material, the method including: a bonding step of bonding a hydrolysate of a silane coupling agent or a condensate of a phosphonic acid derivative to the surface of the base material; and a two-site forming step of polymerizing 2.0 mg/ml or more and 12.0 mg/ml or less of a betaine monomer in presence of a polymerization initiator to form a charge adjustment site and a hydrophilic site after the bonding step.

As compared to the first example, the second example is characterized in that the membrane is formed on the base material by polymerizing 2.0 mg/ml or more and 12.0 mg/ml or less of a betaine monomer in presence of a polymerization initiator to form a charge adjustment site and a hydrophilic site after the bonding step. Constituents that are not described are preferably performed in a same manner as in the first example.

No particular limitation on a polymerization method is provided as long as the betaine monomer can be polymerized under a condition in which a polymerization initiator coexists with the monomer, and a method involving polymerizing the betaine monomer after the polymerization initiator is brought into contact with the base material having been subj ected to the bonding step is preferred from a viewpoint of reactivity. Any method may be used as a method of bringing the polymerization initiator into contact with the base material having been subjected to the bonding step, but a method involving placing the base material having been subjected to the bonding step in a reaction vessel, performing nitrogen purging, and then placing a liquid, in which the polymerization initiator is dissolved is added to a reaction solvent which oxygen has been removed therefrom by nitrogen bubbling, in the reaction vessel, followed by stirring is preferred.

After that, the betaine monomer is dissolved in a reaction solvent. A resultant is placed in the reaction vessel containing the base material and the polymerization initiator, and polymerization is performed by heating. Such polymerization method is preferred. The reaction time (heating time) is preferably 10 minutes or more and 1,440 minutes or less, more preferably 30 minutes or more and 600 minutes or less. As the betaine monomer, those described above may be used. In addition, it is preferred that a concentration of the reaction liquid is adjusted so that a concentration of the betaine monomer becomes 2.0 mg/ml or more and 12.0 mg/ml or less in polymerizing. When the concentration of the monomer falls within the above-mentioned range, the value of B/A can be controlled to 2.0 or more and 5.0 or less, and both low protein adsorptivity and durability can be achieved.

Next, a third example of the production method is described.

The method of producing an antifouling member of the present invention (third example) is a method of producing an antifouling member having a membrane formed on a surface of a base material, the method including: a bonding step of bonding a hydrolysate of a silane coupling agent or a condensate of a phosphonic acid derivative to the surface of the base material; a charge adjustment site forming step of polymerizing a betaine monomer in presence of a polymerization initiator after the bonding step; and a hydrophilic site forming step of adding and polymerizing a hydrophilic monomer to form a hydrophilic site after the charge adjustment site forming step.

As compared to the second example, the third example is characterized in that the charge adjustment site forming step of polymerizing a betaine monomer in presence of a polymerization initiator is performed after the bonding step, and a hydrophilic monomer is added and polymerized to form a hydrophilic site after the charge adjustment site forming step. Constituents that are not described are preferably performed in a same manner as in the first example.

No particular limitation on a polymerization method is provided as long as the betaine monomer can be polymerized under a condition in which the polymerization initiator coexists with the monomer, and a method involving polymerizing the betaine monomer after the polymerization initiator is brought into contact with the base material having been subj ected to the bonding step is preferred from a viewpoint of reactivity. Any method may be used as a method of bringing the polymerization initiator into contact with the base material having been subjected to the bonding step, and a method involving placing the base material having been subjected to the bonding step in a reaction vessel, performing nitrogen purging, and then placing a liquid, in which the polymerization initiator is dissolved is added to a reaction solvent which oxygen has been removed therefrom by nitrogen bubbling, in the reaction vessel, followed by stirring is preferred.

After that, the betaine monomer is dissolved in a reaction solvent. A resultant is placed in the reaction vessel containing the base material and the polymerization initiator, and polymerization is performed by heating. Such polymerization method is preferred. As the betaine monomer, those described above may be used. A reaction time (heating time) is preferably 10 minutes or more and 1,440 minutes or less, more preferably 30 minutes or more and 600 minutes or less. Thus, the charge adjustment site forming step is performed.

After the charge adjustment site forming step, a hydrophilic monomer is added and polymerized to form a hydrophilic site. The formation of the hydrophilic site may be performed by a same method as in the first example.

In addition, in the present invention, the base material preferably includes stainless steel or glass. A hydroxy group is present on an outermost surface of each of stainless steel and glass, and hence a hydrolysate of a silane coupling agent or a phosphonic acid derivative can be condensed to form a covalent bond. The foregoing is preferred because, as a result of a formation of a covalent bond, good durability can be imparted.

In addition, in the present invention, the bonding site to be bonded to the base material is preferably represented by formula (1-1) or formula (1-2): 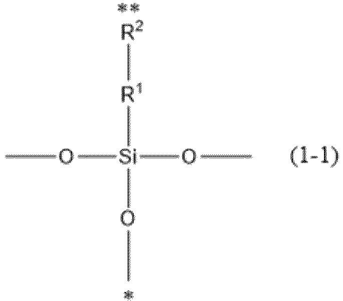 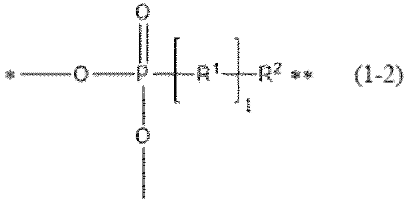 in the formula (1-1) and the formula (1-2), "*" represents a bonding position to the base material, "**" represents a bonding position to the charge adjustment site, R¹ represents any one of an alkylene group or an oxyalkylene group, and R² represents any one of CH=CH (vinylene group), CH=C(CH₃) (isopropenylene group), HC=CH=C(=O)-O (acryloyloxy group), HC=C(CH₃)-C(=O)-O (methacryloyloxy group), or HC=C(CH₃)-C(=O)-NH (methacryloylamino group), and "1" satisfies 1≤1≤6.

It is preferred that the charge adjustment site is a copolymerized product of monomers represented by formula (2-1), formula (2-2), and formula (2-3), or be represented by formula (2-4): 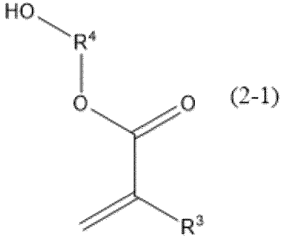 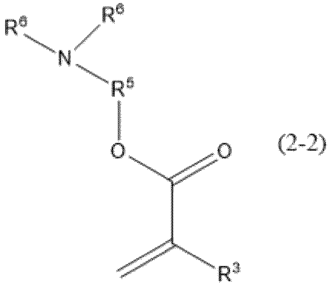 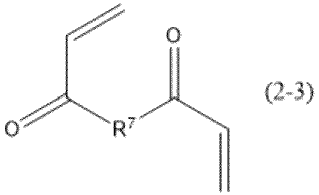 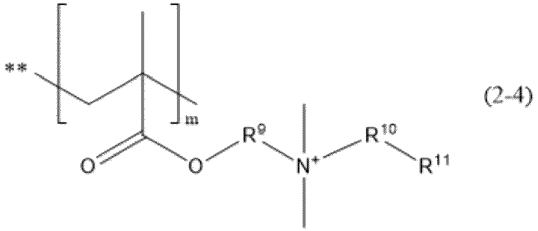 in the formula (2-1), the formula (2-2), and the formula (2-3), R³ represents hydrogen or a methyl group, R⁴, R⁵, and R⁸ each independently represent an alkylene group having 1 to 6 carbon atoms, R⁶ represents hydrogen or an alkyl group having 1 to 3 carbon atoms, and R⁷ represents an oxyalkylene group having 1 to 3 carbon atoms, and in the formula (2-4), "**" represents a bonding position to the bonding site, R⁹ and R¹⁰ each independently represent any one of an alkylene group or an oxyalkylene group, R¹¹ represents -COO⁻, and "m" satisfies 4≤m≤1,000.

The hydrophilic site is preferably represented by formula (3-1) or formula (3-2): 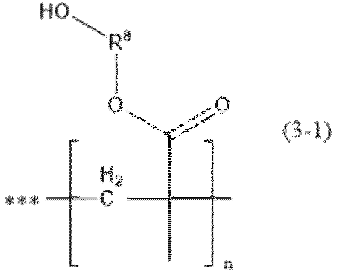 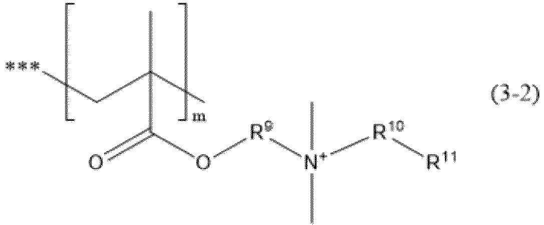 in the formula (3-1) and the formula (3-2), "***" represents a bonding position to the charge adjustment site, R⁸ represents an alkylene group having 1 to 6 carbon atoms, "n" satisfies 4≤n≤1,000, R⁹ and R¹⁰ each independently represent any one of an alkylene group or an oxyalkylene group, R¹¹ represents -COO⁻, and "m" satisfies 4≤m≤1,000.

A thickness of the membrane in the present invention in drying is preferably 1 nm or more and 200 nm or less, more preferably 1 nm or more and less than 10 nm. The thickness of the membrane may be measured by any method, and may be measured with, for example, an ellipsometer. When the thickness of the membrane falls within the above-mentioned ranges, both low protein adsorptivity and durability can be achieved.

### [Application Example]

An application example of the present invention is directed to a member for an in-vitro diagnostic apparatus. As a member for an in-vitro diagnostic apparatus of the present invention, the above-mentioned antifouling member is preferably used. A cell for specimen test analysis and a distal end portion of a pipette are described below as examples of the member for an in-vitro diagnostic apparatus, but the application example of the present invention is not limited to these two applications.

### (Cell for Specimen Test Analysis)

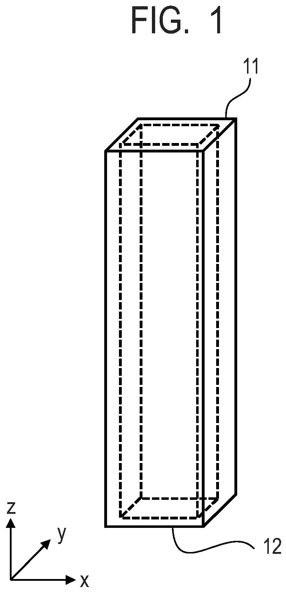

A cell for specimen test analysis has a rectangular tube shape. FIG. 1 is a perspective view of the cell for specimen test analysis in the application example of the present invention. As illustrated in FIG. 1, the cell for specimen test analysis is a glass container having a bottom surface and four side surfaces, and includes a mouth portion 11 (opening portion) on a side opposite to a bottom surface 12.

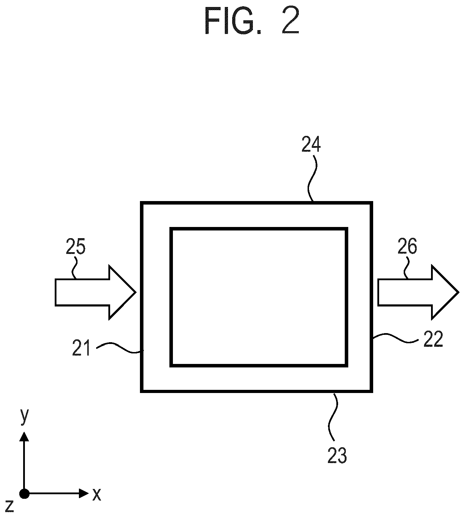

FIG. 2 is a top view of the cell for specimen test analysis in the application example of the present invention. The cell for specimen test analysis has a first optical path surface to which analysis light is input and a second optical path surface opposed to the first optical path surface. Specifically, as illustrated in FIG. 2, the four side surfaces include a first optical path surface 21 to which analysis light 25 is input, a second optical path surface 22 which is opposed to the first optical path surface 21 and from which analysis light 26 is output, a non-optical path surface 23 through which analysis light does not pass, and a non-optical path surface 24 opposed to the non-optical path surface 23. Although the two optical path surfaces are distinguished from each other as the first optical path surface 21 and the second optical path surface 22, such distinction is described merely for the sake of description, and the functions, materials, etc. of the first optical path surface 21 and the second optical path surface 22 are the same. In FIG. 2, the cell for specimen test analysis having a rectangular tube shape is illustrated as an example, but the cell for specimen test analysis may have a cylindrical shape.

In addition, size of the cell for specimen test analysis is not particularly limited. One example is a rectangular shape having inner dimensions of 4 mm×5 mm and outer dimensions of 6 mm×7 mm in a plane parallel to the bottom surface and having a total height (distance from the mouth portion to the bottom surface on an outer side) of 40 mm. There is no problem regarding whether inner dimensions and outer dimensions are larger or smaller than the foregoing. However, in recent years, there is a demand for measurement with a smaller amount of a specimen in order to reduce the burden on a patient, and the cell for specimen test analysis tends to be reduced in size.

The shape, size, etc. of the cell for specimen test analysis are examples and are not limited to those described above. In addition, no problem is caused even when the outer surface of the cell is chamfered or rounded (round surface). When the inner surface has a rectangular tube shape, no problem is caused by rounding the inner surface (round surface).

The cell for specimen test analysis is made of borosilicate glass. As a material of the cell for specimen test analysis, a glass material having high transmittance and being excellent in mechanical strength and chemical durability may be used. In a cleaning step for the cell for specimen test analysis in a specimen test analysis device, an acidic or alkaline cleaning agent is generally used in order to remove contaminants such as protein. When repeated use is assumed, the cell for specimen test analysis is to be exposed to an acid or alkali environment for a long period of time. When the cell for specimen test analysis is made of a resin, the resin is inferior to a glass-based material in terms of chemical durability. Thus, a surface composition of the inner surface of the cell for specimen test analysis is liable to be altered, and the wettability of water thereon may be changed over time. As a material excellent in durability to an acid and an alkali, there are given, for example, quartz glass and borosilicate glass.

As described above, when the cell for specimen test analysis is made of glass, the antifouling member of the present invention can be applied to the cell for specimen test analysis. Through absorbance measurement using the cell for specimen test analysis, a concentration of a substance to be detected (biological substance such as protein or ions) can be detected with high accuracy.

### (Distal End Portion of Pipette)

The distal end portion of a pipette refers to a distal end portion of a pipette tip when the pipette includes the pipette tip, and refers to a distal end portion of a syringe when the pipette does not include the pipette tip. The pipette is electrically conductive at least at the distal end portion thereof. The distal end portion of the pipette is electrically connected to a measurement portion having electrical characteristics. In addition, the pipette can quantitatively perform suction and discharge of a liquid by the reciprocating motion of a plunger. With this configuration, the pipette can provide a liquid to a flow path and collect the liquid therefrom. From a viewpoint of preventing mixing of impurities and the like, it is preferred that the pipette tip be replaceable.

A material of the pipette is not particularly limited. Examples of the material of the pipette include a resin and glass. Polypropylene is generally used as the material of the pipette. The method of imparting conductivity to the distal end portion of the pipette is not particularly limited. For example, the pipette tip made of a resin may contain an additive for imparting conductivity or may include a conductive portion made of a metal. Examples of the additive for imparting conductivity include: carbon-based additives formed of conductive carbon, such as carbon fibers and carbon black; and metal-based additives in which fibers, powder, glass fibers, etc. are each coated with a metal.

When the distal end portion of the pipette contains a metal as described above, the antifouling member of the present invention can be applied to the distal end portion of the pipette. When the distal end portion of the pipette contains a metal, the presence or amount of a substance to be detected (protein, DNA, etc.) can be measured with high accuracy through use of, for example, surface plasmon resonance.

### (Dispensing Probe)

A dispensing probe has a narrow tube shape. A sample dispensing probe performs dispensing involving sucking a sample in a sample vessel and ejecting the sample into a reaction vessel, and a reagent dispensing probe performs dispensing involving sucking a reagent in a reagent vessel and ejecting the reagent into the reaction vessel. A material of the dispensing probe is not particularly limited, but examples of the material include stainless steel, glass, and titanium. In this case, the antifouling member of the present invention can be applied to the dispensing probe.

### Examples

The present invention is more specifically described by way of Examples described below. However, the present invention is by no means limited by the examples. All of the terms "part(s)" and "%" in Examples and in Comparative Examples are on a mass basis unless otherwise stated.

### (Example 1)

A glass slide manufactured by Matsunami Glass Ind., Ltd. (S1111) was placed in a tall beaker containing a 1 M sodium hydroxide aqueous solution and subjected to ultrasonic cleaning, followed by washing with water. 3-(Methacryloyloxy)propyltrimethoxysilane was diluted to 1 mass% with dilute hydrochloric acid (pH=4) and stirred for 1 hour to perform hydrolysis. The hydrolyzed solution was placed in a tall beaker. A glass slide having been subjected to UV/O₃ cleaning for 15 minutes after the ultrasonic cleaning was immersed in the hydrolyzed solution for 1 minute and taken out, followed by drying at 30°C for 5 minutes. The glass slide was washed with pure water to remove an excess hydrolysate, and the washed glass slide was placed in an oven at 90°C to be calcinated for 60 minutes. Thus, a condensation reaction was performed.

The glass slide having undergone the condensation reaction was placed in a 300 ml glass container, and the glass container was covered with a lid. An inside of the glass container was purged with nitrogen, and 100 ml of ultrapure water was placed in the glass container with nitrogen flowing at 200 ml/min. A needle was placed in an ultrapure water in the glass container, and bubbling was performed with nitrogen at 150 ml/min for 30 minutes. 126 mg of 2,2'-azobis[N-(2-carboxyethyl)-2-methylpropionamidine] n-hydrate serving as a polymerization initiator was dissolved in 20 ml of nitrogen-purged ultrapure water, and a resultant was loaded into the 300 ml glass container. After that, the resultant was stirred at room temperature for 60 minutes. 900 mg of 3-[[2-(methacryloyloxy)ethyl]dimethylammonio]propionate serving as a betaine monomer was dissolved in 30 ml of nitrogen-purged ultrapure water, and a resultant was loaded into the 300 ml glass container. After that, a polymerization reaction was performed at 60°C for 4 hours. Then, the glass slide having undergone the polymerization reaction was subjected to pouring and washing with RO water and ultrasonic cleaning at 28 kHz to provide a glass slide having a membrane.

A resultant glass slide having a membrane was subjected to zeta potential measurement, TOF-SIMS measurement, and ESCA measurement by the methods described above. In addition, low protein adsorptivity and durability were evaluated by the following methods.

### <Low Protein Adsorptivity>

Slide & Chamber (2 wells) partitioned by 400 mm² per well is attached to the glass slide having a membrane.

Bovine serum albumin (BSA) is dissolved in an acetate buffer (pH=4.8) having an ionic intensity of 0.001 so as to reach 50 ppm (BSA solution). The 50 ppm BSA solution is diluted with the acetate buffer to prepare samples for calibration curves of 40 ppm, 25 ppm, and 0 ppm.

150 µl of the 50 ppm BSA solution is added to each well. A resultant is covered with a lid and wound with a parafilm. Thus, the BSA solution is incubated at 37°C for 30 minutes.

A solution A and a solution B of Protein Assay BCA Kit (manufactured by FUJIFILM Wako Pure Chemical Corporation) are mixed at a ratio of 50/1 (BCA solution), and 400 µl of the solution is placed in a 1.5 ml sample tube.

After incubation, 90 µl of the sample is taken out from the well and placed in the sample tube containing the BCA solution.

90 µl each of the samples for calibration curves (50 ppm, 40 ppm, 25 ppm, and 0 ppm) is placed in 1.5 ml sample tubes each containing 400 µl of the BCA solution which are separately prepared.

After each of the sample tubes is stirred, a liquid adhering to the lid is removed by centrifugation.

Then, the resultant is kept warm at 60°C for 30 minutes to perform a coloring reaction.

150 µl each of the samples having undergone the coloring reaction is placed in a 96-well plate ("n" number of 3) and measured for absorbance at 562 nm.

An average value of absorbance for an "n" number of 3 is calculated. A calibration curve is created from the average value of absorbance of the samples for calibration curves.

The concentration of the BSA solution in the membrane sample is calculated from the calibration curve, and a BSA adsorption amount (µg) to the membrane is calculated from a change amount from 50 ppm.

The calculated BSA adsorption amount was divided by the area (400 mm²) per well to calculate a BSA adsorption amount (µg/cm²) per unit area. 0.6 µg/cm² that was the BSA adsorption amount to the glass slide was regarded to be a 100% adsorption amount, and the BSA adsorption amount per unit area was divided by 0.6 µg/cm² to calculate a protein adsorption rate. As the BSA adsorption amount of the glass slide, the BSA adsorption amount for an "n" number of 15 or more was calculated in advance, and an average value thereof was used.

A case in which the protein adsorption rate thus calculated is 15% or less is regarded as having sufficient low protein adsorptivity (adsorptivity of protein is low) in the present invention. When the calculated protein adsorption rate is 15% or less, there is no influence of adsorption of the protein on a decrease in detection sensitivity and a decrease in reproducibility.

### <Durability>

An alkaline detergent (Maxpia (trademark) B14 (manufactured by Canon Medical Systems Corporation)) was diluted to 76 times with ultrapure water so as to reach 150 ml.

Maxpia B14 diluted to 76 times and the glass slide having a membrane were placed in a 300 ml flat-bottomed container and stirred for 3 hours.

After 3 hours, the glass slide was taken out and washed with water.

After that, the evaluation of <Low Protein Adsorptivity> was performed to calculate an adsorption rate of a protein.

A case in which durability (protein adsorption rate after washing with water) is 25% or less is regarded as having sufficient durability in the present invention. When the durability (protein adsorption rate after washing with water) is 25% or less, alkali resistance can be effectively exhibited.

### (Example 2)

Processes were performed up to the condensation reaction in a same manner as in Example 1.

The glass slide having undergone the condensation reaction was placed in a 300 ml glass container, and the glass container was covered with a lid. An inside of the glass container was purged with nitrogen, and 65 ml of ultrapure water and 35 ml of ethanol were placed in the glass container with nitrogen flowing at 200 ml/min. A needle was placed in the liquid in the glass container, and bubbling was performed with nitrogen at 150 ml/min for 30 minutes. 126 mg of 2,2'-azobis[N-(2-carboxyethyl)-2-methylpropionamidine] n-hydrate serving as a polymerization initiator was dissolved in 20 ml of nitrogen-purged ultrapure water, and a resultant was loaded into the 300 ml glass container. After that, the resultant was stirred at room temperature for 60 minutes. 900 mg of 2-hydroxyethyl methacrylate serving as a hydrophilic monomer, 100 mg of 2-(dimethylamino)ethyl methacrylate serving as a monomer having polarity opposite to polarity of the base material, and 30 mg of polyethylene glycol diacrylate (n=about 9) serving as a cross-linking agent were dissolved in a mixed solvent of nitrogen-purged ultrapure water and ethanol at a ratio of 10 ml/15 ml, and a resultant was loaded into the 300 ml glass container. After that, a polymerization reaction was performed at 60°C for 3 hours. Further, 1,000 mg of 2-hydroxyethyl methacrylate serving as a hydrophilic monomer to be added was dissolved in a mixed solvent of nitrogen-purged ultrapure water and ethanol at a ratio of 7 ml/7 ml, and a resultant was loaded into the 300 ml glass container. Then, a polymerization reaction was performed at 60°C for 3 hours. After that, the glass slide having undergone the polymerization reaction was subjected to pouring and washing with ethanol and RO water and ultrasonic cleaning at 28 kHz to provide a glass slide having a membrane.

A resultant glass slide was subjected to zeta potential measurement, TOF-SIMS measurement, and ESCA measurement. In addition, low protein adsorptivity and durability were evaluated in a same manner as in Example 1.

### (Examples 3, 5, 6, and 14)

Processes were performed up to the evaluation in a same manner as in Example 1 except that the betaine monomer was changed as shown in Table 2.

**Table 2**

| | Betaine monomer | | |
|---|---|---|---|
| | Kind | Weight (mg) | Concentration (mg/ml) |
| Example 1 | 3-[[2-(Methacryloyloxy)ethyl]dimethy]ammonio]propionate | 900 | 6.0 |
| Example 3 | 3-[[2-(Methacryloyloxy)ethyl]dimethylammonio]propane-1-sulfonate | 900 | 6.0 |
| Example 5 | 3-[[2-(Methacryloyloxy)ethyl]dimethy]ammonio]propionate | 360 | 2.4 |
| Example 6 | 3-[[2-(Methacryloyloxy)ethyl]dimethy]ammonio]propionate | 1,800 | 12.0 |
| Example 14 | 2-(Methacryloyloxy)ethyl 2-(trimethylammonio)ethyl phosphate | 630 | 4.2 |

### (Examples 4, 9, 11, 12, and 15)

Processes were performed up to the evaluation in a same manner as in Example 2 except that the materials were changed as shown in Table 3.

**Table 3**

| | Polymerization initiator | | Hydrophilic monomer | | Monomer having polarity opposite to polarity of base material | | Cross-linking agent | | Hydrophilic monomer to be added | |
|---|---|---|---|---|---|---|---|---|---|---|
| | Kind | Amount (mg) | Kind | Amount (mg) | Kind | Amount (mg) | Kind | Amount (mg) | Kind | Amount (mg) |
| Example 2 | VA-57 | 126 | HEMA | 900 | DMAEM | 100 | EG (n=9) | 30 | HEMA | 1,000 |
| Example 4 | VA-57 | 126 | HEMA | 850 | DMAEM | 150 | EG (n=9) | 30 | HEMA | 1,000 |
| Example 9 | VA-57 | 126 | HEMA | 900 | DMAEM | 100 | EG (n=9) | 30 | CBMA | 450 |
| Example 11 | VA-57 | 126 | HEMA | 920 | DEAEM | 80 | EG (n=9) | 30 | HEMA | 1,000 |
| Example 12 | VA-57 | 126 | HEMA | 900 | DMAEM | 100 | HDDA | 90 | HEMA | 1,000 |
| Example 15 | V50 | 126 | HEMA | 900 | DMAEM | 100 | EG (n=9) | 30 | HEMA | 1,000 |

In Table 3, VA-57 represents 2,2'-azobis[N-(2-carboxyethyl)-2-methylpropionamidine] n-hydrate, V50 represents 2,2'-azobis(2-methylpropionamidine) dihydrochloride, HEMA represents 2-hydroxyethyl methacrylate, CBMA represents 3-[[2-(methacryloyloxy)ethyl]dimethylammonio]propionate, DMAEM represents 2-(dimethylamino)ethyl methacrylate, DEAEM represents 2-(diethylamino)ethyl methacrylate, EG (n=9) represents polyethylene glycol diacrylate (n=about 9), and HDDA represents 1,6-hexanediol diacrylate.

### (Example 7)

Processes were performed up to the condensation reaction in a same manner as in Example 1.

The glass slide having undergone the condensation reaction was placed in a 300 ml glass container, and the glass container was covered with a lid. An inside of the glass container was purged with nitrogen, and 50 ml of ultrapure water and 50 ml of ethanol were placed in the glass container with nitrogen flowing at 200 ml/min. A needle was placed in the liquid in the glass container, and bubbling was performed with nitrogen at 150 ml/min for 30 minutes. 126 mg of 2,2'-azobis[N-(2-carboxyethyl)-2-methylpropionamidine] n-hydrate serving as a polymerization initiator was dissolved in 20 ml of nitrogen-purged ultrapure water, and a resultant was loaded into the 300 ml glass container. After that, the resultant was stirred at room temperature for 60 minutes. 360 mg of 3-[[2-(methacryloyloxy)ethyl]dimethylammonio]propionate serving as a betaine monomer was dissolved in 30 ml of nitrogen-purged ultrapure water, and a resultant was loaded into the 300 ml glass container. After that, a polymerization reaction was performed at 60°C for 3 hours. Further, 720 mg of 2-hydroxyethyl methacrylate serving as a hydrophilic monomer to be added was dissolved in 15 ml of nitrogen-purged ultrapure water, and a resultant was loaded into the 300 ml glass container. Then, a polymerization reaction was performed at 60°C for 3 hours. After that, the glass slide having undergone the polymerization reaction was subjected to pouring and washing with ethanol and RO water and ultrasonic cleaning at 28 kHz to provide a glass slide having a membrane.

A resultant glass slide was subjected to zeta potential measurement, TOF-SIMS measurement, and ESCA measurement. In addition, low protein adsorptivity and durability were evaluated in a same manner as in Example 1.

### (Example 8)

Processes were performed up to the evaluation in a same manner as in Example 7 except that the betaine monomer was changed to 450 mg of 3-[[2-(methacryloyloxy)ethyl]dimethylammonio]propionate and the hydrophilic monomer to be added was changed to 900 mg of polyethylene glycol monomethyl ether methacrylate (average Mn: 950).

### (Example 10)

A glass slide manufactured by Matsunami Glass Ind., Ltd. (S1111) was placed in a tall beaker containing a 1 M sodium hydroxide aqueous solution and subjected to ultrasonic cleaning. 2-Methacryloyloxyethyl acid phosphate was diluted to 0.5 mass% with tetrahydrofuran and placed in a tall beaker. A glass slide having been subjected to UV/O₃ cleaning for 15 minutes after the ultrasonic cleaning was immersed in the diluted 2-methacryloyloxyethyl acid phosphate for 60 minutes and taken out, followed by drying at 30°C for 5 minutes. The glass slide was washed with tetrahydrofuran, and the washed glass slide was placed in an oven at 120°C to be calcinated for 60 minutes. Thus, a condensation reaction was performed.

The glass slide having undergone the condensation reaction was placed in a 300 ml glass container, and the glass container was covered with a lid. An inside of the glass container was purged with nitrogen, and 100 ml of ultrapure water was placed in the glass container with nitrogen flowing at 200 ml/min. A needle was placed in the ultrapure water in the glass container, and bubbling was performed with nitrogen at 150 ml/min for 30 minutes. 126 mg of 2,2'-azobis[N-(2-carboxyethyl)-2-methylpropionamidine] n-hydrate serving as a polymerization initiator was dissolved in 20 ml of nitrogen-purged ultrapure water, and a resultant was loaded into the 300 ml glass container. After that, the resultant was stirred at room temperature for 60 minutes. 630 mg of 3-[[2-(methacryloyloxy)ethyl]dimethylammonio]propionate serving as a betaine monomer was dissolved in 30 ml of nitrogen-purged ultrapure water, and a resultant was loaded into the 300 ml glass container. After that, a polymerization reaction was performed at 60°C for 4 hours. Then, the glass slide having undergone the polymerization reaction was subjected to pouring and washing with RO water and ultrasonic cleaning at 28 kHz to provide a glass slide having a membrane.

The resultant glass slide having a membrane was subjected to zeta potential measurement, TOF-SIMS measurement, and ESCA measurement by the methods described above. In addition, low protein adsorptivity and durability were evaluated by the above methods.

### (Example 13)

Processes were performed up to the condensation reaction in a same manner as in Example 1.

The glass slide having undergone the condensation reaction was placed in a 300 ml glass container, and the glass container was covered with a lid. An inside of the glass container was purged with nitrogen, and 50 ml of ultrapure water and 20 ml of ethanol were placed in the glass container with nitrogen flowing at 200 ml/min. A needle was placed in a liquid in the glass container, and bubbling was performed with nitrogen at 150 ml/min for 30 minutes. 50 mg of 2,2'-azobis(isobutyronitrile) serving as a polymerization initiator was dissolved in 50 ml of nitrogen-purged ethanol, and a resultant was loaded into the 300 ml glass container. After that, the resultant was stirred at room temperature for 60 minutes. 900 mg of 3-[[2-(methacryloyloxy)ethyl]dimethylammonio]propionate serving as a betaine monomer was dissolved in 30 ml of nitrogen-purged ultrapure water, and the resultant was loaded into the 300 ml glass container. After that, a polymerization reaction was performed at 60°C for 4 hours. After that, the glass slide having undergone the polymerization reaction was subjected to pouring and washing with ethanol and RO water and ultrasonic cleaning at 28 kHz to provide a glass slide having a membrane.

The resultant glass slide was subjected to zeta potential measurement, TOF-SIMS measurement, and ESCA measurement. In addition, low protein adsorptivity and durability were evaluated in a same manner as in Example 1.

### (Example 16)

Processes were performed from the preparation up to the evaluation in a same manner as in Example 1 except that a glass slide manufactured by Matsunami Glass Ind., Ltd. (S1111) was changed to a SUS304 plate (width×length×thickness=26 mm×60 mm×1 mm).

### (Example 17)

Processes were performed from the preparation up to the evaluation in a same manner as in Example 2 except that a glass slide manufactured by Matsunami Glass Ind., Ltd. (S1111) was changed to a SUS304 plate (width×length×thickness=26 mm×60 mm×1 mm).

### (Comparative Example 1)

A glass slide manufactured by Matsunami Glass Ind., Ltd. (S1111) was placed in a tall beaker containing a 1 M sodium hydroxide aqueous solution and subjected to ultrasonic cleaning, followed by washing with water. After that, UV/O₃ cleaning was performed for 15 minutes.

Lipidure-S (manufactured by NOF CORPORATION) was diluted to 1 mass% with toluene and placed in a tall beaker. The glass slide after the UV/O₃ cleaning was immersed in diluted Lipidure-S for 1 minute and taken out, followed by drying at 30°C for 24 hours, to form a membrane.

The glass slide having the membrane formed thereon was subjected to zeta potential measurement, TOF-SIMS measurement, and ESCA measurement in a same manner as in Example 1. In addition, low protein adsorptivity and durability were evaluated.

### (Comparative Example 2)

Processes were performed up to the evaluation in a same manner as in Example 2 except that the hydrophilic monomer was changed to 800 mg of 2-hydroxyethyl methacrylate and the monomer having polarity opposite to polarity of the base material was changed to 200 mg of 2-(dimethylamino)ethyl methacrylate.

### (Comparative Example 3)

Processes were performed up to the condensation reaction in a same manner as in Example 1.

A glass slide having undergone the condensation reaction was placed in a 300 ml glass container, and the glass container was covered with a lid. An inside of the glass container was purged with nitrogen, and 65 ml of ultrapure water and 35 ml of ethanol were placed in the glass container with nitrogen flowing at 200 ml/min. A needle was placed in the liquid in the glass container, and bubbling was performed with nitrogen at 150 ml/min for 30 minutes. 126 mg of 2,2'-azobis[N-(2-carboxyethyl)-2-methylpropionamidine] n-hydrate serving as a polymerization initiator was dissolved in 20 ml of nitrogen-purged ultrapure water, and a resultant was loaded into the 300 ml glass container. After that, the resultant was stirred at room temperature for 60 minutes. 900 mg of 2-hydroxyethyl methacrylate serving as a hydrophilic monomer was dissolved in a mixed solvent of nitrogen-purged ultrapure water and ethanol at a ratio of 10 ml/15 ml, and the resultant was loaded into the 300 ml glass container. After that, a polymerization reaction was performed at 60°C for 6 hours. After that, the glass slide having undergone the polymerization reaction was subjected to pouring and washing with ethanol and RO water and ultrasonic cleaning at 28 kHz to provide a glass slide having a membrane.

The resultant glass slide was subjected to zeta potential measurement, TOF-SIMS measurement, and ESCA measurement. In addition, low protein adsorptivity and durability were evaluated in a same manner as in Example 1.

### (Comparative Example 4)

A glass slide manufactured by Matsunami Glass Ind., Ltd. (S1111) was placed in a tall beaker containing a 1 M sodium hydroxide aqueous solution and subjected to ultrasonic cleaning, followed by washing with water. After that, UV/O₃ cleaning was performed for 15 minutes.

LAMBIC-1000W (manufactured by OSAKA ORGANIC CHEMICAL INDUSTRY LTD.) was diluted to 5 times with ultrapure water and placed in a tall beaker. The glass slide after the UV/O₃ cleaning was immersed in the diluted LAMBIC-1000W for 1 minute and taken out, followed by drying at 80°C for 15 minutes, to form a membrane.

The glass slide having the membrane formed thereon was subjected to zeta potential measurement, TOF-SIMS measurement, and ESCA measurement in a same manner as in Example 1. In addition, low protein adsorptivity and durability were evaluated.

### (Comparative Example 5)

A glass slide manufactured by Matsunami Glass Ind., Ltd. (S1111) was placed in a tall beaker containing a 1 M sodium hydroxide aqueous solution and subjected to ultrasonic cleaning, followed by washing with water. After that, UV/O₃ cleaning was performed for 15 minutes.

The cleaned glass slide was placed in a vacuum desiccator containing 10 drops of (3-aminopropyl)triethoxysilane. A system (in a vacuum desicator) was depressurized to 1 mbar or less and placed under reduced pressure for 30 minutes after being disconnected from a pump. After that, the glass slide was calcinated at 110°C for 30 minutes under normal pressure. The calcinated glass slide was placed in a reaction vessel, and nitrogen purging was performed. 50 ml of dehydrated tetrahydrofuran, 1.3 ml of 2-bromoisobutyryl bromide, and 1.5 ml of triethylamine were added to the reaction vessel and stirred for 1 hour. The glass slide was taken out from the reaction vessel and washed with tetrahydrofuran, methanol, and ultrapure water, followed by drying.

The dried glass slide and 143 mg of copper(I) bromide were placed in a reaction vessel, and nitrogen purging was performed. 156 mg of 2,2'-bipyridyl and 874 mg of 3-[[2-(methacryloyloxy)ethyl]dimethylammonio]propionate were placed in a pear-shaped flask, and nitrogen purging was performed. Then, 5 ml of nitrogen-purged methanol and 5 ml of nitrogen-purged ultrapure water were added to the pear-shaped flask to dissolve it. The solution in the pear-shaped flask was placed in the reaction vessel containing the glass slide and the copper(I) bromide through use of a syringe and stirred for 1 hour. After that, a resultant was washed with ethanol, phosphate buffered saline (PBS), and ultrapure water to provide a glass slide having a polymer brush formed thereon.

The resultant glass slide having a polymer brush formed thereon was subjected to zeta potential measurement, TOF-SIMS measurement, and ESCA measurement. In addition, low protein adsorptivity and durability were evaluated in a same manner as in Example 1.

### (Comparative Example 6)

Processes were performed up to the evaluation in a same manner as in Example 1 except that the amount of 3-[[2-(methacryloyloxy)ethyl]dimethylammonio]propionate was changed to 270 mg.

### (Comparative Example 7)

Processes were performed up to the condensation reaction in a same manner as in Example 1.

A glass slide having undergone a condensation reaction was placed in a 300 ml glass container, and the glass container was covered with a lid. An inside of the glass container was purged with nitrogen, and 65 ml of ultrapure water and 35 ml of ethanol were placed in the glass container with nitrogen flowing at 200 ml/min. A needle was placed in the liquid in the glass container, and bubbling was performed with nitrogen at 150 ml/min for 30 minutes. 126 mg of 2,2'-azobis[N-(2-carboxyethyl)-2-methylpropionamidine] n-hydrate serving as a polymerization initiator was dissolved in 20 ml of nitrogen-purged ultrapure water, and a resultant was loaded into the 300 ml glass container. After that, the resultant was stirred at room temperature for 60 minutes. 900 mg of 2-hydroxyethyl methacrylate serving as a hydrophilic monomer, 100 mg of 2-(dimethylamino)ethyl methacrylate serving as a monomer having polarity opposite to polarity of the base material, and 30 mg of polyethylene glycol diacrylate (n=about 9) serving as a cross-linking agent were dissolved in a mixed solvent of nitrogen-purged ultrapure water and ethanol at a ratio of 10 ml/15 ml, and the resultant was loaded into the 300 ml glass container. After that, a polymerization reaction was performed at 60°C for 3 hours. The glass slide having undergone the polymerization reaction was subjected to pouring and washing with ethanol and RO water and ultrasonic cleaning at 28 kHz to provide a glass slide having a membrane.

A resultant glass slide was subjected to zeta potential measurement, TOF-SIMS measurement, and ESCA measurement. In addition, low protein adsorptivity and durability were evaluated in a same manner as in Example 1.

### (Comparative Example 8)

A surface of a SUS304 plate (width×length×thickness=26 mm×60 mm×1 mm) was washed with acetone and dried. The SUS304 plate was immersed in a 2 mass% aqueous solution of 3-(acryloyloxy)propyltrimethoxysilane (with 2 mass% acetic acid added) for 10 minutes and taken out, followed by drying at 40°C for 24 hours. After that, the SUS304 plate was washed with water and then washed with acetone. The treated SUS304 plate was immersed in a solution of 1.5 mass% azobisisobutyronitrile in ethanol for 5 minutes and taken out, followed by drying.

The SUS304 plate was placed in a glass container containing a 1.25 M aqueous solution of a 3-sulfopropyl methacrylate potassium salt, and the glass container was covered with a lid.

After purging was performed by introducing nitrogen gas, the glass container was immersed in a water bath at 60°C for 5 hours to perform heating and polymerization. After that, a surface was washed with water to wash off unreacted monomers, etc. to provide a SUS304 plate having a membrane. The resultant SUS304 plate having a membrane was subjected to zeta potential measurement, TOF-SIMS measurement, and ESCA measurement, and low protein adsorptivity and durability were evaluated in a same manner as in Example 1.

### (Comparative Example 9)

The process was performed up to the evaluation in a same manner as in Example 1 except that an amount of 3-[[2-(methacryloyloxy)ethyl]dimethylammonio]propionate was changed to 180 mg.

Physical properties and evaluation results of the membranes obtained in Examples 1 to 17 and Comparative Examples 1 to 9 are shown in Table 4.

**Table 4**

| | Zeta potential (mV) of membrane at pH of 7 | Halogen atom ion amount/total ion amount | B/A | Low protein adsorptivity | Durability |
|---|---|---|---|---|---|
| Example 1 | -5.9 | 0.01 | 3.7 | 0% | 12% |
| Example 2 | -2.7 | 0.01 | 4.3 | 0% | 13% |
| Example 3 | -9.3 | 0.01 | 2.9 | 11% | 23% |
| Example 4 | 9.5 | 0.01 | 4.6 | 15% | 25% |
| Example 5 | -1.6 | 0.01 | 2.2 | 13% | 25% |
| Example 6 | -5.3 | 0.01 | 4.9 | 15% | 24% |
| Example 7 | -5.1 | 0.01 | 3.2 | 15% | 24% |
| Example 8 | -9.3 | 0.02 | 2.8 | 10% | 22% |
| Example 9 | -4.3 | 0.01 | 3.1 | 6% | 17% |
| Example 10 | -5.3 | 0.01 | 2.8 | 5% | 18% |
| Example 11 | -4.2 | 0.01 | 3.3 | 6% | 15% |
| Example 12 | -3.1 | 0.01 | 3.0 | 7% | 19% |
| Example 13 | -5.2 | 0.01 | 3.6 | 0% | 23% |
| Example 14 | -3.2 | 0.01 | 2.7 | 1% | 17% |
| Example 15 | -5.7 | 0.02 | 3.2 | 4% | 24% |
| Example 16 | -2.1 | 0.01 | 3.0 | 0% | 17% |
| Example 17 | -1.5 | 0.01 | 3.8 | 8% | 20% |
| Comparative Example 1 | -6.7 | 0.01 | 4.5 | 24% | 95% |
| Comparative Example 2 | 12.1 | 0.01 | 3.9 | 77% | 100% |
| Comparative Example 3 | -11.8 | 0.01 | 2.3 | 33% | 100% |
| Comparative Example 4 | -12.4 | 0.01 | 3.4 | 22% | 75% |
| Comparative Example 5 | -0.4 | 0.03 | 3.2 | 0% | 100% |
| Comparative Example 6 | -1.9 | 0.01 | 1.9 | 35% | 100% |
| Comparative Example 7 | 1.9 | 0.01 | 1.7 | 21% | 100% |
| Comparative Example 8 | -4.2 | 0.01 | 10.5 | 0% | 100% |
| Comparative Example 9 | -11.1 | 0.01 | 1.6 | 35% | 100% |

The antifouling member of the present invention can achieve both low protein adsorptivity and durability. Thus, when being used as a member for an in-vitro diagnostic apparatus, the antifouling member of the present invention enables measurement with high accuracy and can be used repeatedly.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An antifouling member comprising a membrane formed on a base material, wherein the membrane contains a condensate of a hydrolysate of a silane coupling agent or a condensate of a phosphonic acid derivative,
wherein the membrane does not contain a halogen atom,
wherein the membrane has a zeta potential of -10.0 mV or more and 10.0 mV or less in water having a pH of 7, and
wherein the antifouling member has a value of B/A of 2.0 or more and 5.0 or less, where A represents a ratio of an amount of C atom (atomic%) to an amount of atom of an element X (atomic%) contained in a surface of the base material in a largest amount in X-ray photoelectron spectroscopy analysis (ESCA measurement) of the base material, and B represents a ratio of an amount of C atom (atomic%) to an amount of atom of the element X (atomic%) in X-ray photoelectron spectroscopy analysis (ESCA measurement) of the membrane on the base material.

2. The antifouling member according to claim 1, wherein the membrane contains a copolymer of a monomer having polarity opposite to polarity of the base material, a hydrophilic monomer, and a cross-linking agent.

3. The antifouling member according to claim 1, wherein the membrane contains a polymer of a betaine monomer.

4. The antifouling member according to claim 1, wherein the membrane contains a polymer of a betaine monomer and a polymer of a hydrophilic monomer.

5. The antifouling member according to claim 2 or 4, wherein the hydrophilic monomer is electrically neutral.

6. The antifouling member according to claim 2 or 4, wherein the hydrophilic monomer is at least one kind selected from the group consisting of 2-hydroxyethyl methacrylate; N-vinyl-2-pyrrolidone; and polyethylene glycol monomethyl ether methacrylate.

7. The antifouling member according to claim 3 or 4, wherein the betaine monomer is at least one kind selected from the group consisting of 2-(methacryloyloxy)ethyl 2-(trimethylammonio)ethyl phosphate; 2-[[2-(methacryloyloxy)ethyl]dimethylammonio]acetate; 3-[[2-(methacryloyloxy)ethyl]dimethylammonio]propionate; 3-[(3-acrylamidopropyl)dimethylammonio]propanoate; 3-[[2-(methacryloyloxy)ethyldimethylammonio]propane- 1 -sulfonate; 4-[[2-(methacryloyloxy)ethyl]dimethylammonio]butane-1-sulfonate; 3-[[2-(acryloyloxy)ethyl]dimethylammonio]propane-1-sulfonate; 3-[(3-methacrylamidopropyl)dimethylammonio]propane-1-sulfonate; and 3-[(3-acrylamidopropyl)dimethylammonio]propane-1-sulfonate.

8. The antifouling member according to any one of claims 1 to 7,
wherein the silane coupling agent is at least one kind selected from the group consisting of: 3-(methacryloyloxy)propyltrimethoxysilane; 3-(acryloyloxy)propyltrimethoxysilane; 3-(methacryloyloxy)propyltriethoxysilane; 3-(acryloyloxy)propyltriethoxysilane; vinyltrimethoxysilane; triethoxyvinylsilane; allyltrimethoxysilane; allyltriethoxysilane; and (triethoxysilyl)methyl methacrylate, and
wherein the phosphonic acid derivative is at least one kind selected from the group consisting of: 2-methacryloyloxyethyl acid phosphate; 2-acryloyloxyethyl acid phosphate; 2-(methacryloyloxy)ethyl phosphate; and acid phosphoxy polyethylene glycol monomethacrylate.

9. The antifouling member according to claim 2, wherein the monomer having polarity opposite to polarity of the base material is an amine monomer.

10. The antifouling member according to claim 9, wherein the amine monomer is at least one kind selected from the group consisting of: 2-(dimethylamino)ethyl methacrylate; 2-(dimethylamino)ethyl acrylate; 2-(diethylamino)ethyl methacrylate; 2-(diisopropylamino)ethyl methacrylate; and 2-(tert-butylamino)ethyl methacrylate.

11. The antifouling member according to claim 2, wherein the cross-linking agent is at least one kind selected from the group consisting of: ethylene glycol diacrylate; ethylene glycol dimethacrylate; polyethylene glycol diacrylate; polyethylene glycol dimethacrylate; and 1,6-hexanediol diacrylate.

12. The antifouling member according to any one of claims 1 to 11, wherein a material of the base material includes stainless steel or glass.

13. The antifouling member according to any one of claims 1 to 12, wherein the membrane includes a charge adjustment site having polarity opposite to polarity of the base material and a hydrophilic site.

14. The antifouling member according to claim 13,
wherein a bonding site bonded to the base material is represented by the following formula (1-1) or the following formula (1-2),
wherein the charge adjustment site is a copolymerized product of monomers represented by the following formula (2-1), the following formula (2-2), and the following formula (2-3), or is represented by the following formula (2-4), and
wherein the hydrophilic site is represented by the following formula (3-1) or the following formula (3-2): 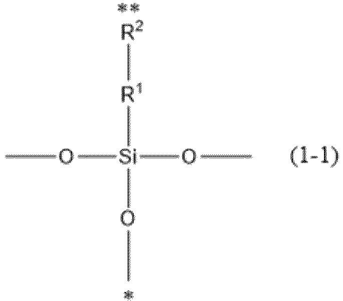 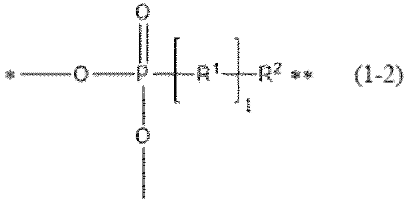 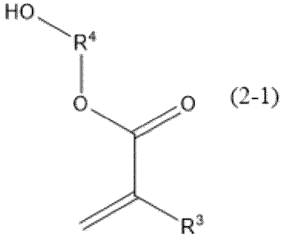 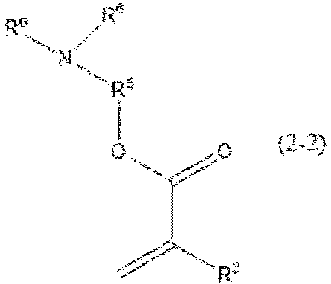 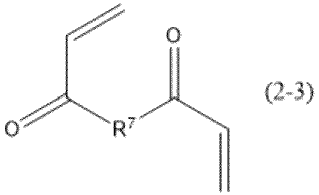 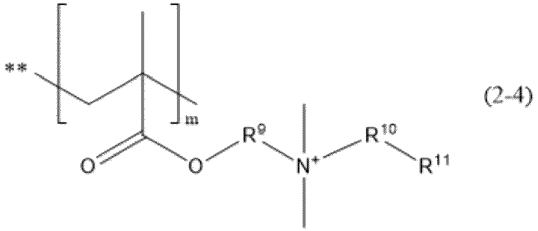 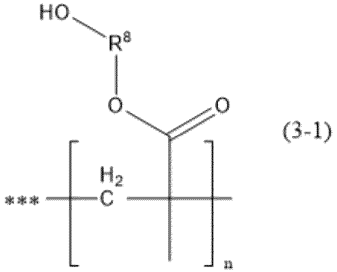 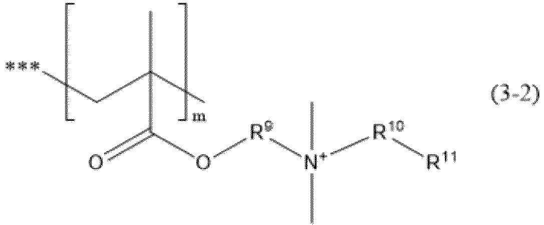
in the formula (1-1) and the formula (1-2), "*" represents a bonding position to the base material, "**" represents a bonding position to the charge adjustment site, R¹ represents any one of an alkylene group or an oxyalkylene group, and R² represents any one of CH=CH (vinylene group), CH=C(CH₃) (isopropenylene group), HC=CH=C(=O)-O (acryloyloxy group), HC=C(CH₃)-C(=O)-O (methacryloyloxy group), or HC=C(CH₃)-C(=O)-NH (methacryloylamino group), and "l" satisfies 1≤l≤6;
in the formula (2-1), the formula (2-2), and the formula (2-3), R³ represents hydrogen or a methyl group, R⁴, R⁵, and R⁸ each independently represent an alkylene group having 1 to 6 carbon atoms, R⁶ represents hydrogen or an alkyl group having 1 to 3 carbon atoms, and R⁷ represents an oxyalkylene group having 1 to 3 carbon atoms;
in the formula (2-4), "**" represents a bonding position to the bonding site, R⁹ and R¹⁰ each independently represent any one of an alkylene group or an oxyalkylene group, R¹¹ represents -COO⁻, and "m" satisfies 4≤m≤1,000; and
in the formula (3-1) and the formula (3-2), "***" represents a bonding position to the charge adjustment site, R⁸ represents an alkylene group having 1 to 6 carbon atoms, "n" satisfies 4≤n≤1,000, R⁹ and R¹⁰ each independently represent any one of an alkylene group or an oxyalkylene group, R¹¹ represents -COO⁻, and "m" satisfies 4≤m≤1,000.

15. An in-vitro diagnostic apparatus using the antifouling member according to any one of claims 1 to 14.

16. A method of producing an antifouling member having a membrane formed on a surface of a base material, the method comprising:
a bonding step of bonding a hydrolysate of a silane coupling agent or a condensate of a phosphonic acid derivative to the surface of the base material;
a charge adjustment site forming step of copolymerizing a monomer having polarity opposite to polarity of the base material, a hydrophilic monomer, and a cross-linking agent in presence of a polymerization initiator after the bonding step; and
a hydrophilic site forming step of adding and polymerizing a hydrophilic monomer to form a hydrophilic site after the charge adjustment site forming step.
